# EUROPEAN PATENT APPLICATION

(11) **EP 1 022 336 A1**
(43) Date of publication of application: **26.07.2000**
(21) Application number: 98945639.7
(22) Date of filing: 06.10.1998
(51) Int. Cl.: C12N 15/12, C07K 14/435, C12N 15/63, C12N 5/10, C12P 21/02, C07K 16/18, C12P 21/08, A61K 38/17

(54) **POLYPEPTIDE, cDNA ENCODING THE SAME, AND USE OF THEM**

(30) Priority: 07.10.1997 JP 27467397
(71) Applicant: ONO PHARMACEUTICAL CO., LTD., Osaka-shi, Osaka 541-8526 (JP)
(72) Inventor: HONJO, Tasuku, Kyoto-shi, Kyoto 606-0001 (JP); KATO, Keizo, Shingu-shi, Wakayama 647-0014 (JP); TADA, Hideaki, Minase Res. Inst., ONO Phar. Co.,, Mishima-gun, Osaka 618-8585 (JP)
(74) Representative: Henkel, Feiler, Hänzel
(86) International application number: JP9804515
(87) International publication number: WO9918205

(57) **Abstract**

A polypeptide prepared from mouse ES cell strains by the SST method and a homologous polypeptide obtained from mouse kidney, human uterus; mouse fetus, human fetal liver and pancreas libraries; a cDNA encoding the polypeptide; a fragment selectively hybridizing with the sequence of the cDNA; a replication or expression plasmid containing the cDNA integrated thereinto; a host cell transformed with the plasmid; an antibody against the polypeptide; and a pharmaceutical composition containing the polypeptide or the antibody.

## Description

### Technical Field

The present invention relates to novel polypeptides, a method for preparation of them, a cDNA encoding it, a vector containing it, a host cell transformed with the vector, an antibody against the peptide, and a pharmaceutical composition containing the polypeptide or the antibody.

More particularly, the present invention relates to novel polypeptides produced by a certain kind of mouse ES cell strains, homologues thereof, a process for the preparation of them, cDNAs encoding the said polypeptides, a vector containing the polypeptide, a host cell transformed by the vector, antibody against the said polypeptide, a pharmaceutical composition containing the polypeptide or antibody.

### Technical Background

In the process of embriogenesis, innert cell mass possessing multiple differentiation activity at the stage of blastocyst are allowed to differentiate into various cell lineages such as hematopoietic cells, muscle and bone etc., going through mesoderm induction. It is considered that the differentiation into each cell lineage is controlled depending on the surrounding of the said cell itself i.e., strength, combination and variation of stimulation by differentiation inducing factors (or inhibitory factors) or the cellsurface molecules. Transcription factors specific for each cell lineage or intracellular proteins involved in signal transduction system also play an important role in the differentiation. It is no exaggeration, however, to say that the substance, that essentially controls expression of the factors and consequent fate of the cell, are transmembrane proteins (receptor) and their ligand molecules. Some molecules belonging to TGF-b super family or FGF family such as activine have been already identified as the substance. In addition to an investigation on the mechanism of the functions, research for unknown molecules involved in the functions has been focused. Efforts to isolate the molecules that function at the early stage of the embriogenesis and allow the cell to commit to an cell lineage have been performed.

For example, it has been reported that using a differential display method and subtraction method can isolate genes specifically expressing at the early stage of the embryo or each portion of the embryo, i.e., endoderm, mesoderm and ectoderm (See M. J. Guimaraes et. al., Development, 121, 3335-3346, 1995 and S.. M. Harrison et al., Development, 121, 2479-2489, 1995). However, these methods require much quantity of small section of embryo at early stage of embriogenesis. In addition, although a gene was isolated by using these methods, it often happened to encode an intercellular protein and consequently neither candidates of soluble factors nor receptors have been yet isolated.

The present inventors et al. have studied cloning method to isolate genes encoding proliferation and/or differentiation factors, that are involved in hematopoietic systems and immune systems. Focusing their attention on the fact that most of the secretory proteins such as proliferation and/or differentiation factors (for example, various cytokines etc.) and transmembrane proteins such as receptors thereof have unique peptide sequences called as the signal sequence at their N-termini, the present inventors et al. have conducted extensive studies on methodology to efficiently and selectively isolate a gene encoding for the signal sequence. Finally, the present inventors et al. have successfully developed a screening method for the signal sequence (signal sequence trap: SST) by effective amplification of the DNA region encoding N-termini (See Japanese Patent Application Kokai Hei 6-315380, Tashiro et al, Science, 261, 600-603, 1993).

### Disclosure of the present invention

The present inventors et al. have diligently performed certain investigation in order to isolate novel secretory proteins using SST method, that are expressed in the course of the differentiation of ES cells and are involved in early stage of embriogenesis and hemopoiesis.

Eventually, using a culturing system that allows ES cells, that are derived from innert cell mass at the stage of blastocyst possessing a multiple differentiation activity, to differentiate into hematocyte (See T. Nakano, et al., Science, 265, 1098-1101, 1994) in combination with SST method to efficiently isolate secretary proteins or membrane proteins, the present inventors et al. have successfully discovered a new factor (polypeptide) named as OHP106 and cDNA encoding it, in addition, new factors related to OHP106 and cDNAs encoding them and then have completed the present invention.

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequence and the nucleotide sequence of mouse OHP106 of the present invention. It was also indicated from the hydrophobisity analysis that the polypeptides of the present invention had no transmembrane region. Taken altogether, it was proved that polypeptides of the present invention were new secretary proteins.

That is to say, the invention relates to:
(1) a polypeptide comprising an amino acid sequence shown in SEQ ID NOS. 1, 4, 7, 10 or 13,
(2) a cDNA encoding the polypeptides described above (1),
(3) a cDNA comprising a nucleotide sequence shown in SEQ ID NOS. 2, 5, 8, 11 or 14,
(4) a cDNA comprising a nucleotide sequence shown in SEQ ID NOS. 3, 6, 9, 12 or 15.

### Brief Description of the Drawing

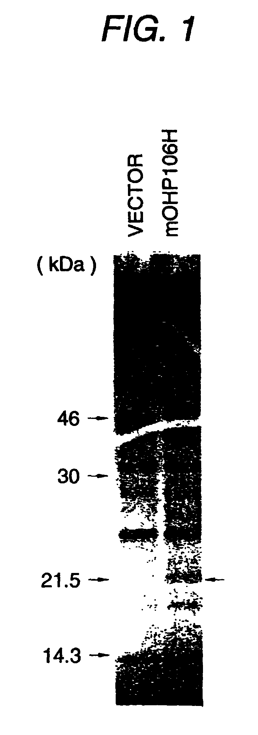

Fig. 1 shows a printout illustrating expression of mouse OHP106 protein detected by imaging-analyzer (FUJI BAS2000) on the acrylamido gel after electrophoresis (SDS-PAGE).

### Detailed Description of the present invention

The present invention relates to a polypeptide comprising amino acid sequence shown in SEQ ID NOS. 1, 4, 7, 10 or 13 in substantially purified form, a homologue thereof, a fragment of the sequence and a homologue of the fragment.

Further, the present invention relates to cDNAs encoding the above peptides. More particularly the invention is provided cDNAs comprising nucleotide sequence shown in SEQ ID NOS. 2, 5, 8, 11 or 14 or sequence shown in SEQ ID NOS. 3, 6, 12 or 15, and cDNA containing a fragment which is selectively hybridizing to the cDNA comprising nucleotide sequence shown in SEQ ID NOS. 2, 5, 8, 11 or 14 or nucleotide sequence shown in SEQ ID NOS. 3, 6, 9, 12 or 15.

A said cDNA capable for hybridizing to the cDNA includes the contemporary sequence of the above sequence.

A polypeptide comprising amino acid sequence shown in SEQ ID NOS. 1, 4, 7, 10 or 13 in substantially purified form will generally comprise the polypeptide in a preparation in which more than 90%, e.g. 95%, 98% or 99% of the polypeptide in the preparation is that of the SEQ ID NOS. 1, 4, 7, 10 or 13.

A homologue of polypeptide comprising amino acid sequence shown in SEQ ID NOS. 1, 4, 7, 10 or 13 will be generally at least 70%, preferably at least 80 or 90% and more preferably at least 95% homologous to the polypeptide comprising the said amino acid sequence over a region of at least 20, preferably at least 30, for instance 40, 60 or 100 more contiguous amino acids. Such a polypeptide homologue will be referred to a polypeptide of the present invention.

Generally, a fragment of polypeptide comprising amino acid sequence shown in SEQ ID NOS. 1, 4, 7, 10 or 13 or its homologues will be at least 10, preferably at least 15, for example 20, 25, 30, 40, 50 or 60 amino acids in length.

A cDNA capable of selectively hybridizing to the cDNA comprising nucleotide sequence shown in SEQ ID NOS. 2, 5, 8, 11 or 14 or nucleotide sequence shown in SEQ ID NOS. 3, 6, 9, 12 or 15 will be generally at least 70%, preferably at least 80 or 90%, and more preferably at least 95% homologous to the cDNA comprising the said nucleotide sequence over a region of at least 20, preferably at least 30, for instance 40, 60 or 100 or more contiguous nucleotides. Such a cDNA will be referred to "a cDNA of the present invention".

Fragments of the cDNA comprising nucleotide sequence shown in SEQ ID NOS. 2, 5, 8, 11 or 14 or nucleotide sequence shown in SEQ ID NOS. 3, 6, 9, 12 or 15 will be at least 10, preferably at least 15, for example 20, 25, 30 or 40 nucleotides in length, and will be also referred to "a cDNA of the present invention" as used herein.

A further embodiment of the present invention provides replication and expression vectors carrying cDNA of the present invention. The vectors may be, for example, plasmid, virus or phage vectors provided with an origin of replication, optionally a promoter for the expression of the said cDNA and optionally a regulator of the promoter. The vector may contain one or more selectable marker genes, for example a ampicillin resistance gene. The vector may be used in vitro, for example of the production of RNA corresponding to the cDNA, or used to transfect a host cell.

A further embodiment of the present invention provides host cells transformed with the vectors for the replication and expression of the cDNA of the present invention, including the cDNA comprising nucleotide sequence shown in SEQ ID NOS. 2, 5, 8, 11 or 14 or nucleotide sequence shown in SEQ ID NOS. 3, 6, 9, 12 or 15 or the open reading frame thereof. The cells will be chosen to be compatible with the vector and may for example be bacterial, yeast, insect cells or mammalian cells.

A further embodiment of the present invention provides a method of producing a polypeptide which comprises culturing host cells of the present invention under conditions effective to express a polypeptide of the present invention. Preferably, in addition, such a method is carried out under conditions in which the polypeptide of the present invention is expressed and then produced from the host cells.

cDNA of the present invention may also be inserted into the vectors described above in an antisense orientation in order to proved for the production of antisense mRNA. Such antisense mRNA may be used in a method of controlling the levels of a polypeptide of the present invention in a cell.

The invention also provides monoclonal or polyclonal antibodies against a polypeptide of the present invention. The invention further provides a process for the production of monoclonal or polyclonal antibodies to the polypeptides of the present invention. Monoclonal antibodies may be prepared by common hybridoma technology using polypeptides of the present invention or fragments thereof, as an immunogen. Polyclonal antibodies may also be prepared by common means which comprise inoculating host animals, (for example a rat or a rabbit etc.), with polypeptides of the present invention and recovering immune serum.

The present invention also provides pharmaceutical compositions containing a polypeptide of the present invention, or an antibody thereof, in association with a pharmaceutically acceptable diluent and/or carrier.

The polypeptide of the present invention specified in (1) includes that which a part of their amino acid sequence is lacking (e.g., a polypeptide comprised of the only essential sequence for revealing a biological activity in an amino acid sequence shown in SEQ ID NOS. 1, 4, 7, 10 or 13), that which a part of their amino acid sequence is replaced by other amino acids (e.g., those replaced by an amino acid having a similar property) and that which other amino acids are added or inserted into a part of their amino acid sequence, as well as those comprising the amino acid sequence shown in SEQ ID NOS. 1, 4, 7, 10 or 13.

As known well, there are one to six kinds of codon as that encoding one amino acid (for example, one kind of codon for Methionine (Met), and six kinds of codon for Leucine (Leu) are known). Accordingly, the nucleotide sequence of cDNA can be changed in order to encode the polypeptide having the same amino acid sequence.

The cDNA of the present invention, specified in (2) includes a group of every nucleotide sequence encoding polypeptides (1) shown in SEQ ID NOS. 1, 4, 7, 10 or 13. There is a probability that yield of a polypeptide is improved by changing a nucleotide sequence.

The cDNA specified in (3) is the embodiment of the cDNA shown in (2), and indicate the sequence of natural form.

The cDNA shown in (4) indicates the sequence of the cDNA specified in (3) with natural non-translational region.

cDNA carrying nucleotide sequence shown in SEQ ID NOS. 3, 6, 9, 12 or 15 is prepared by the following method:

Preparation of cDNA library for signal sequence trap (SST) is as follows:
(1) mRNA is isolated from the targeted cells, second-strand cDNA synthesis is performed by using random primer with certain restriction enzyme (enzyme I) recognition site,
(2) fractionated in size, double-strand cDNA is ligated to adapter containing certain restriction endonuclease (enzyme II) recognition site, differ from enzyme I,
(3) reacting a primer containing enzyme I recognition site with a primer containing enzyme II recognition site by polymerase series reaction (abbreviated as PCR hereinafter), amplified cDNA is digested with enzyme I - enzyme II, is fractionated again,
(4) obtained cDNA fragment is inserted into eukaryotic cell expression vector on the upstream region of invertase gene which signal sequence is deleted and the library was transformed.

### Detailed description of each step is as follows:

In step (1), total mRNA is isolated from target cell lines according to the method of Chomcynski, P et al. (Anl. Biochem. 162, 156 (1987)). mRNA is purified with Oligo(dT) column etc.

As for target cell, mouse ES (Embryonic Stem) D3 cell line (T. C. Doetschman, et. al., J. Embryol. Exp. Morphol., 87, 27, 1985) which is obtained by culturing together with stroma OP9 cell line derived from op/op mouse neonatal calvaria (H. Kodama, et. al., Exp. Hematol, 22, 979-984, 1994) and by differentiation and/or inducing into hematopoietic cell is chosen.

A single strand cDNA is prepared by using a random primer ligated with (enzyme I) recognition site and then a double strand cDNA is prepared by the method of Gublerr & Hoffman.

In step (2), cDNA is created blunt-ends with T4 cDNA polymerase, ligated enzyme II adapter and analyzed with agarose-gel electrophoresis and is selected cDNA fraction ranging in size from 400 to 600 bp. Any sites may be used as restriction endonuclease recognition site I which is linked to adapter and restriction endonuclease recognition site II which is used in step (2), if both sites are different each other. Preferably, SalI is used as enzyme I and EcoRI as enzyme II.

In step (3), PCR is carried out in order to amplify cDNA. The cDNA thus amplified is digested with enzyme I and II and is subjected electrophoresis on an agarose gel to fractionated into fragmented cDNAs of 300 to 800 bp.

In step (4), transformation is performed. Gene of known membrane protein etc. (it is called as reporter gene) in which signal sequence is deleted and cDNA fragment obtained in step (3) on the upstream region of the said gene are inserted to a plasmid vector for expression in eukaryotic cell. Many kinds of vectors are known as eukaryotic cell expression plasmid vector. For example, pcDL-SRα or pcEV-4 is used.

For reporter gene, many kinds of gene of mature protein part of soluble secretory protein and membrane protein are used. As for reporter gene used, it is necessary to confirm the expression of the said gene by some method, for example, antibody method etc. In the present invention, gene of human IL-2α receptor is used. Many host Escherichia coli strains for transformation are known, any strain may be used, DH5 competent cell is preferable. Transformant is cultured by known methods to obtain cDNA library of the present invention.

In the process for constructing a cDNA library according to the present invention, there is a high possibility that gene fragments encoding for the protein of N-termini are contained in the library. However, not every clone contains signal peptide. Further, not all of the gene fragments encode for unknown (novel) signal sequences. Therefore, it is necessary to screen a gene fragment encoding for an unknown signal sequence from said library.

Namely, the cDNA library is divided into small pools of an appropriate size and integrated into an expression system. Examples of the expression system for producing a polypeptide include mammalian cells (for example, monkey COS-7 cells, Chinese hamster CHO cells, mouse L cells etc.). Transfection may be performed in accordance with DEAE-dextran method. After the completion of the incubation, the expression of the reporter gene is examined.

It is known that a reporter gene would be expressed even though the signal sequence is the one characteristic to another secretory protein. That is to say, the fact that the reporter gene has been expressed indicates that a signal sequence of same secretory protein has been integrated into the library. Positive pools are further divided into smaller ones and the expression and the judgement are repeated until a single clone is obtained. The expression of the reporter gene can be judged by, for example, fluorescence-labeled antibody assay, enzyme-linked immunosorbent assay (ELISA) or radio-immuno assay (RIA), depending on kinds of the employed reporter gene. In the present invention, fluorescence-labeled antibody assay using fluorescen · isothiocyanate labeled anti-human Tac is used.

Next, the nucleotide sequence of the positive clone which has been isolated is determined and compared with the sequence registered in data base. In case of a cDNA which is proved to encode an unknown protein, the full length clone can be isolated by hybridizing with full length cDNA library or genoma library using the said cDNA fragment as a probe, or by PCR using synthesized oligo nucleotide containing an adequate sequence from cDNA library or mRNA derived from mammalian cells to determine the full length nucleotide sequence.

Once the nucleotide sequences of the positive clone are determined partially or preferably fully, it is possible to obtain a cDNA encodes mammalian protein of the present invention itself homologue or subset. cDNA encoding the said mammalian protein can be obtained by PCR using synthesized oligo nucleotide containing an adequate sequence from cDNA library or mRNA derived from mammalian cell, or by hybridizing with cDNA library or genoma library using a fragment of the said mouse nucleotide sequence as a probe.

For example, after nucleotide sequence of mouse OHP106 shown in the SEQ ID NOS. 2 or 3 was determined, mouse OHP106K, which was the clone having 22 bp insert sequence in the open reading frame of mouse OHP106, and human OHP106, which was about 550 bp length and which was considered to be human counterpart of mouse OHP106 because of high homology to the nucleotide sequence of mouse OHP106, were discovered in the mouse kidney cDNA library and human uterine cDNA library, respectively. In addition, mouse OHP106H and human OHP106H, which were the clone having a significant homology to the nucleotide sequence of mouse OHP106, were discovered in mouse fetus aged 13.5 days and 14.5 days cDNA library, and human fetus liver and pancreas cDNA library, respectively.

If a cDNA obtained above contains the nucleotide sequence of cDNA fragment obtained by SST (or consensus sequence thereof), it will be thought that the cDNA encodes signal sequence. So it is clear that the cDNA will be full-length or almost full. (All signal sequences exist at N-termini of a protein and are encoded at 5'-temini of open reading frame of cDNA.)

The confirmation may be carried out by Northern analysis with the said cDNA as a probe. It is thought that the cDNA is almost complete length, if length of the cDNA is almost the same length of the mRNA obtained in the hybridizing band.

Once the nucleotide sequences shown in SEQ ID NOS. 2, 5, 8, 11 or 14 or nucleotide sequences shown in SEQ ID NOS. 3, 6, 9, 12 or 15 are determined, cDNAs of the present invention are obtained by chemical synthesis, or by hybridization making use of nucleotide fragments which are chemically synthesized as a probe. Furthermore, cDNAs of the present invention are obtained in desired amount by transforming a vector that contains the cDNA into a proper host, and culturing the transformant.

The polypeptides of the present invention may be prepared by:
(1) isolating and purifying from an organism or a cultured cell,
(2) chemically synthesizing, or
(3) using recombinant cDNA technology,
preferably, by the method described in (3) in an industrial production.

Examples of expression system (host-vector system) for producing a polypeptide by using recombinant cDNA technology are the expression systems of bacteria, yeast, insect cells and mammalian cells.

In the expression of the polypeptide, for example, in E. Coli, the expression vector is prepared by adding the initiation codon (ATG) to 5' end of a cDNA encoding mature peptide, connecting the cDNA thus obtained to the downstream of a proper promoter (e.g., trp promoter, lac promoter, λ PL promoter, T7 promoter etc.), and then inserting it into a vector (e.g., pBR322, pUC18, pUC19 etc.) which functions in an E. coli strain.

Then, an E. coli strain (e.g., E. coli DH1 strain, E. coli JM109 strain, E. coli HB101 strain, etc.) which is transformed with the expression vector described above may be cultured in a appropriate medium to obtain the desired polypeptide. When a signal sequence of bacteria (e.g., signal sequence of pel B) is utilized, the desired polypeptide may be also released in periplasm. Furthermore, a fusion protein with other polypeptide may be also produced readily.

In the expression of the polypeptide, for example, in a mammalian cells, for example, the expression vector is prepared by inserting the cDNA encoding nucleotide shown in SEQ ID NOS. 3, 6, 9 or 12 into the downstream of a proper promoter (e.g., SV40 promoter, LTR promoter, metallothionein promoter etc.) in a proper vector (e.g., retrovirus vector, papilloma virus vector, vaccinia virus vector, SV40 vector, etc.). A proper mammalian cell (e.g., monkey COS-7 cell, Chinese hamster CHO cell, mouse L cell etc.) is transformed with the expression vector thus obtained, and then the transformant is cultured in a proper medium to get a desired polypeptide on the cell membrane. The polypeptide available by the way described above can be isolated and purified by conventional biochemical method.

### Industrial Applicability

It is considered that the polypeptide of the present invention and a cDNA which encodes the polypeptide will show one or more of the effects or biological activities (including those which relates to the assays cited below) The effects or biological activities described in relation to the polypeptide of the present invention are provided by administration or use of the polypeptide or by administration or use of a cDNA molecule which encodes the polypeptide (e.g., vector suitable for gene therapy or cDNA introduction).

### [Cytokine activity and cell proliferation/differentiation activity]

The protein of the present invention may exhibit cytokine, cell proliferation (either inducing or inhibiting) or cell differentiation (either inducing or inhibiting) activity or may induce production of other cytokines in certain cell populations. Many protein factors discovered to date, including all known cytokines, have exhibited activity in one or more factor dependent cell proliferation assays, and hence the assays serve as a convenient confirmation of cytokine activity. The activity of a polypeptide of the present invention is evidenced by any one of a number of routine factor dependent cell proliferation assays for cell lines.

### [Immune stimulating/suppressing activity]

The protein of the present invention may also exhibit immune stimulating or immune suppressing activity. The protein of the present invention may be useful in the treatment of various immune deficiencies and disorders (including severe combined immunodeficiency (SCID)), e.g., in regulating (up or down) growth and proliferation of T and/or B lymphocytes, as well as effecting the cytolytic activity of NK cells and other cell populations. These immune deficiencies may be genetic or be caused by viral infection such as HIV as well as bacterial or fungal infections, or may result from autoimmune disorders. More specifically, infectious diseases causes by viral, bacterial, fungal or other infection may be treatable using the polypeptide of the present invention, including infections by HIV, hepatitis viruses, herpes viruses, mycobacteria, leshmania, malaria and various fungal infections such as candida Of course, in this regard, the protein of the present invention may also be useful where a boost to the immune system generally would be indicated, i.e., in the treatment of cancer.

The protein of the present invention may be useful in the treatment of allergic reactions and conditions, such as asthma or other respiratory problems. The protein of the present invention may also be useful in the treatment of the other conditions required to suppress the immuno system (for example, asthma or respiratory disease.)

The protein of the present invention may also suppress chronic or acute inflammation, such as, for example, that associated with infection such as septic shock or systemic inflammatory response syndrome (SIRS), inflammatory bowel disease, Crohn's disease or resulting from over production of cytokines such as TNF or IL-I wherein the effect was demonstrated by IL- 11.

### [Hematopoiesis regulating activity]

The protein of the present invention may be useful in regulation of hematopoiesis and, consequently, in the treatment of myeloid or lymphoid cell deficiencies. Even marginal biological activity in support of colony forming cells or of factor-dependent cell lines indicates involvement in regulating hematopoiesis. The said biological activities are concerned with the following all or some example(s). e.g. in supporting the growth and proliferation of erythroid progenitor cells alone or in combination with other cytokines, thereby indicating utility, for example, in treating various anemias or for use in conjunction with irradiation/chemotherapy to stimulate the production of erythroid precursors and/or erythroid cells; in supporting the growth and proliferation of myeloid cells such as granulocytes and monocytes/macrophages (i.e., traditional CSF activity) useful, for example, in conjunction with chemotherapy to prevent or treat consequent myelo-suppression; in supporting the growth and proliferation of megakaryocytes and consequently of platelets thereby allowing prevention or treatment of various platelet disorders such as thrombocytopenia, and generally for use in place of or complimentary to platelet transfusions; and/or in supporting the growth and proliferation of hematopoietic stem cells which are capable of maturing to any and all of the above-mentioned hematopoietic cells and therefore find therapeutic utility in various stem cell disorders (such as those usually treated with transplantation, including, without limitation, aplastic anemia and paroxysmal nocturnal hemoglobinuria), as well as in repopulating the stem cell compartment post irradiation/chemotherapy, either in-vitro or ex-vivo (i.e. in conjunction with bone marrow transplantation) as normal cells or genetically manipulated for gene therapy.

The activity of the protein of the present invention may, among other means, be measured by the following methods:

### [Tissue generation/regeneration activity]

The protein of the present invention also may have utility in compositions used for bone, cartilage, tendon, Ligament and/or nerve tissue growth or regeneration, as well as for wound healing and tissue repair, and in the treatment of burns, incisions and ulcers.

The protein of the present invention, which induces cartilage and/or bone growth in circumstances where bone is not normally formed, may be applied to the healing of bone fractures and cartilage damage or defects in humans and other animals. Such a preparation employing the protein of the present invention may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery.

The protein of the present invention may also be used in the treatment of periodontal disease, and in other tooth repair processes. Such agents may provide an environment to attract bone-forming cells, stimulate growth of bone-forming cells or induce differentiation of progenitors of bone-forming cells. The protein of the present invention may also be useful in the treatment of osteoporosis or osteoarthritis, such as through stimulation of bone and/or cartilage repair or by blocking inflammation or processes of tissue destruction (collagenase activity, osteoclast activity, etc.) mediated by inflammatory processes.

Another category of tissue regeneration activity that may be attributable to the protein of the present invention is tendon/ligament formation. The protein of the present invention, which induces tendon/ligament-like tissue or other tissue formation in circumstances where such tissue is not normally formed, may be applied to the healing of tendon or ligament tears, deformities and other tendon or ligament detects in humans and other animals. Such a preparation employing the protein inducing a tendon/Ligament-like tissue may have prophylactic use in preventing damage to tendon or ligament tissue, as well as use in the improved fixation of tendon or ligament to bone or other tissues, and in repairing defects to tendon or ligament tissue. De novo tendon/ligament-like tissue formation induced by a composition of the present invention contributes to the repair of congenital, trauma induced, or other tendon or ligament defects of other origin, and is also useful in cosmetic plastic surgery for attachment or repair of tendons or ligaments. The compositions of the present invention may provide an environment to attract tendon- or ligament-forming cells, stimulate growth of tendon- or ligament-forming cells, induce differentiation of progenitors of tendon- or ligament-forming cells, or induce growth of tendon Ligament cells or progenitors ex vivo for return in vivo to effect tissue repair. The compositions of the present invention may also be useful in the treatment of tendinitis, Carpal tunnel syndrome and other tendon or ligament defects. The compositions may also include an appropriate matrix and/or sequestering agent as a carrier as is well known in the art.

The protein of the present invention may also be useful for proliferation of neural cells and for regeneration of nerve and brain tissue. i.e. for the treatment of central and peripheral nervous system diseases and neuropathies. as well as mechanical and traumatic disorders, which involve degeneration, death or trauma to neural cells or nerve tissue. More specifically, the protein of the present invention may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotrophic lateral sclerosis, and Shy-Drager syndrome. Further conditions which may be treated in accordance with the invention include mechanical and traumatic disorders, such as spinal cord disorders, head trauma and cerebrovascular diseases such as stroke. Peripheral neuropathies resulting from chemotherapy or other medical therapies may also be treatable using the polypeptide of the present invention.

It is expected that the protein of the present invention may also exhibit activity for generation of other tissues, such as organs (including, for example, pancreas, liver, intestine, kidney, skin, endothelium), muscle (smooth, skeletal or cardiac) and vascular (including vascular endothelium) tissue, or for promoting the proliferation of cells comprising such tissues. Part of the desired effects may be by inhibition of fibrotic scarring to allow normal tissue to regenerate.

The protein of the present invention may also be useful for gut protection or regeneration and treatment of lung or liver fibrosis, reperfusion injury in various tissues, and conditions resulting from systemic cytokine damage.

### [Activin/Inhibin activity]

The protein of the present invention may also exhibit activin- or inhibin-related activities. Inhibins are characterized by their ability to inhibit the release of follicle stimulating hormone (FSH), while activins are characterized by their ability to stimulate the release of follicle stimulating hormone (FSH). Thus, the protein of the present invention alone or in heterodimers with a member of the inhibin *a family, may be useful as a contraceptive based on the ability of inhibins to decrease fertility in female mammals and decrease spermatogenesis in male mammals. Administration of sufficient amounts of other inhibins can induce infertility in these mammals.
Alternatively, the protein of the present invention, as a homodimer or as a heterodimer with other protein subunits of the inhibin-*b group, may be useful as a fertility inducing therapeutic, based upon the ability of activin molecules in stimulating FSH release from cells of the anterior pituitary (See USP 4,798,885).

The protein of the present invention may also be useful for advancement of the onset of fertility in sexually immature mammals, so as to increase the lifetime reproductive performance of domestic animals such as cows, sheep and pigs.

### [Chemotactic/chemokinetic activity]

The protein of the present invention may have chemotactic or chemokinetic activity e.g., functioning as a chemokine, for mammalian cells, including, for example, monocytes, neutrophils, T-cells, mast cells, eosinophils and/or endothelial cells. Chemotactic and chemokinetic proteins can be used to mobilize or attract a desired cell population to a desired site of action. Chemotactic or chemokinetic proteins provide particular advantages in treatment of wounds and other trauma to tissues, as well as in treatment of localized infections. For example, attraction of lymphocytes, monocytes or neutrophils to tumors or sites of infection may result in improved immune responses against the tumor or infecting agent.

If a protein or peptide can stimulate, directly or indirectly, the directed orientation or movement of such cell population, it has chemotactic activity for a particular cell population. Preferably, the protein or peptide has the ability to directly stimulate directed movement of cells. Whether a particular protein has chemotactic activity for a population of cells can be readily determined by employing such protein or peptide in any known assay for cell chemotaxis.

### [Hemostatic and thrombolytic activity]

The protein of the present invention may also exhibit hemostatic or thrombolyic activity. As a result, such a protein is expected to be useful in treatment of various coagulation disorders (including hereditary disorders, such as hemophilias) or to enhance coagulation and other hemostatic events in treating wounds resulting from trauma, surgery or other causes. A protein of the present invention may also be useful for dissolving or inhibiting formation of thromboses and for treatment and prevention of conditions resulting therefrom such as, for example, infarction or stroke.

### [Receptor/ligand activity]

The protein of the present invention may also demonstrate activity as receptors, receptor ligands or inhibitors or agonists of receptor/ligand interactions. Examples of such receptors and ligands include, without limitation, cytokine receptors and their ligands, receptor kinases and their ligands, receptor phosphatases and their ligands, receptors involved in cell-cell interactions and their ligands (including cellular adhesion molecules such as Selectins, Integrins and their ligands) and receptor/ligand pairs involved in antigen presentation, antigen recognition and development of cellular and humoral immune responses. Receptors and ligands are also useful for screening of potential peptide or small molecule inhibitors of the relevant receptor/ligand interaction. The protein of the present invention (including, without limitation, fragments of receptors and ligands) may themselves be useful as inhibitors of receptor/ligand interactions.

### [Other activity]

The protein of the present invention may also exhibit one or more of the following additional activities or effects: inhibiting growth of or killing the infecting agents including bacteria, viruses, fungi and other parasites; effecting (suppressing or enhancing) body characteristics including height, weight, hair color, eye color, skin, other tissue pigmentation, or organ or body part size or shape such as, for example, breast augmentation or diminution etc.; effecting elimination of dietary fat, protein, carbohydrate; effecting behavioral characteristics including appetite, libido, stress, cognition (including cognitive disorders), depression and violent behaviors; providing analgesic effects or other pain reducing effects; promoting differentiation and growth of embryonic stem cells in lineages other than hematopoietic lineages; in the case of enzymes, correcting deficiencies of the enzyme and treating deficiency-related diseases.

The protein with above activities, is suspected to have following functions by itself or interaction with its ligands or receptors or association with other molecules. For example, proliferation or cell death of B cells, T cells and/or mast cells; specific induction by promotion of class switch of immunoglobulin genes; differentiation of B cells to antibody-forming cells; proliferation, differentiation, or cell death of precursors of granulocytes; proliferation, differentiation, or cell death of precursors of monocytes-macrophages; proliferation, of up regulation or cell death of neutrophils, monocytes-macrophages, eosinophils and/or basophils; proliferation, or cell death of precursors of megakaryocytes; proliferation, differentiation, or cell death of precursors of neutrophils; proliferation, differentiation, or cell death of precursors of T cells and B cells; promotion of production of erythrocytes; sustainment of proliferation of erythrocytes, neutrophils, eosinophils, basophils, monocytes-macrophages, mast cells, precursors of megakaryocyte; promotion of migration of neutrophils, monocytes-macrophages, B cells and/or T cells; proliferation or cell death of thymocytes; suppression of differentiation of adipocytes; proliferation or cell death of natural killer cells; proliferation or cell death of hematopoietic stem cells; suppression of proliferation of stem cells and each hematopoietic precursor cells; promotion of differentiation from mesenchymal stem cells to osteoblasts or chondrocytes, proliferation or cell death of mesenchymal stem cells, osteoblasts or chondrocytes and promotion of bone absorption by activation of osteoclasts and promotion of differentiation from monocytes to osteoclasts.

The polypeptide of the present invention is also suspected to function to nervous system, so expected to have functions below; differentiation to kinds of neurotransmitter-responsive neurons, survival or cell death of these cells; promotion of proliferation or cell death of glial cells; spread of neural dendrites; survival or cell death of gangriocytes; proliferation, promotion of differentiation, or cell death of astrocytes; proliferation, survival or cell death of peripheral neurons; proliferation or cell death of Schwann cells; proliferation, survival or cell death of motoneurons.

Furthermore, in the process of development of early embryonic, the polypeptide of the present invention is expected to promote or inhibit the organogenesis of epidermis, brain, backbone, and nervous system by induction of ectoderm, that of notochord connective tissues (bone, muscle, tendon), hemocytes, heart, kidney, and genital organs by induction of mesoderm, and that of digestive apparatus (stomach, intestine, liver, pancreas), respiratory apparatus (lung, trachea) by induction of endoderm. In adult, also, this polypeptide is thought to proliferate or inhibit the above organs.

Therefore, the polypeptide of the present invention itself is expected to be used as an agent for the prevention or treatment of disease of progression or suppression of immune, nervous, or bone metabolic function, hypoplasia or overgrowth of hematopoietic cells: for example, inflammatory disease (rheumatism, ulcerative colitis, etc.), decrease of hematopoietic stem cells after bone marrow transplantation, decrease of leukocytes, platelets, B-cells, or T-cells after radiation exposure or chemotherapeutic dosage against cancer or leukemia, anemia, infectious disease, cancer, leukemia, AIDS, bone metabolic disease (osteoporosis etc.), various degenerative disease (Alzheimer's disease, multiple sclerosis, etc.), or nervous lesion.

In addition, since the polypeptide of the present invention is thought to induce the differentiation or growth of organs derived from ectoderm, mesoderm, and endoderm, this polypeptide is expected to be an agent for tissue repair (epidermis, bone, muscle, tendon, heart, kidney, stomach, intestine, liver, pancreas, lung, and trachea, etc.).

By using polyclonal or monoclonal antibodies against the polypeptide of the present invention, quantitation of the said polypeptide in the body can be performed. It can be used in the study of relationship between this polypeptide and disease or diagnosis of disease, and so on. Polyclonal and monoclonal antibodies can be prepared using this polypeptide or its fragment as an antigen by conventional methods.

Identification, purification or molecular cloning of known or unknown proteins which bind the polypeptide of the present invention (preferably polypeptide of extracellular domain) can be performed using the polypeptide of the present invention by, for example, preparation of the affinity-column.

Identification of the downstream signal transmission molecules which interact with the polypeptide of the present invention in cytoplasma and molecular cloning of the gene can be performed:
by west-western method using the polypeptide of the present invention (preferably polypeptide of transmembrane region or intracellular domain) or
by yeast two-hybrid system using the cDNA (preferably cDNA encoding transmembrane region or cytoplasmic domain of the polypeptide).

Agonists/antagonists of this receptor polypeptide and inhibitors between receptor and signal transduction molecules can be screened using the polypeptide of the present invention.

cDNAs of the present invention are useful not only the important and essential template for the production of the polypeptide of the present invention which is expected to be largely useful, but also be useful for diagnosis or therapy (for example, treatment of gene lacking, treatment to stop the expression of the polypeptide by antisense cDNA (mRNA)). Genomic cDNA may be isolated with the cDNA of the present invention, as a probe. As the same manner, a human gene encoding which can be highly homologous to the cDNA of the present invention, that is, which encodes a polypeptide highly homologous to the polypeptide of the present invention and a gene of animals excluding mouse which can be highly homologous to the cDNA of the present invention, also may be isolated.

### [Application to Medicaments]

The polypeptide of the present invention or the antibody specific for the polypeptide of the present invention is administered systemically or topically and in general orally or parenterally, preferably parenterally, intravenously and intraventricularly, for preventing or treating the said diseases.

The doses to be administered depend upon age, body weight, symptom, desired therapeutic effect, route of administration, and duration of the treatment etc. In human adults, one dose per person is generally between 100 µg and 100 mg, by oral administration, up to several times per day, and between 10 µg and 100 mg, by parental administration up to several times per day.

As mentioned above, the doses to be used depend upon various conditions. Therefore, there are cases in which doses lower than or greater than the ranges specified above may be used.

The compounds of the present invention, may be administered as solid compositions, liquid compositions or other compositions for oral administration, as injections, liniments or suppositories etc. for parental administration.

Solid compositions for oral administration include compressed tablets, pills, capsules, dispersible powders, and granules. Capsules include soft or hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate, etc.). The compositions may also comprise, as is normal practice, additional substances other than inert diluents: e.g. lubricating agents (such as magnesium stearate etc.), disintegrating agents (such as cellulose calcium glycolate, etc.), stabilizing agents (such as human serum albumin, lactose etc.), and assisting agents for dissolving (such as arginine, asparaginic acid etc.).

The tablets or pills may, if desired, be coated with a film of gastric or enteric materials (such as sugar, gelatin, hydroxypropyl cellulose or hydroxypropylmethyl cellulose phthalate, etc.), or be coated with more than two films. And then, coating may include containment within capsules of absorbable materials such as gelatin.

Liquid compositions for oral administration include pharmaceutically-acceptable emulsions, solutions, syrups and elixirs. In such compositions, one or more of the active compound(s) is or are contained in inert diluent(s) commonly used (purified water, ethanol etc.). Besides inert diluents, such compositions may also comprise adjuvants (such as wetting agents, suspending agents, etc.), sweetening agents, flavoring agents, perfuming agents, and preserving agents.

Other compositions for oral administration include spray compositions which may be prepared by known methods and which comprise one or more of the active compound(s). Spray compositions may comprise additional substances other than inert diluents: e.g. stabilizing agents (sodium sulfite etc.), isotonic buffer (sodium chloride, sodium citrate, citric acid, etc.). For preparation of such spray compositions, for example, the method described in the United States Patent No. 2,868,691 or 3,095,355 (herein incorporated in their entireties by reference) may be used.

Injections for parental administration include sterile aqueous or non-aqueous solutions, suspensions and emulsions. In such compositions, one or more active compound(s) is or are admixed with at least one inert aqueous diluent(s) (distilled water for injection, physiological salt solution, etc.) or inert non-aqueous diluents(s) (propylene glycol, polyethylene glycol, olive oil, ethanol, POLYSOLBATE 80 (Trade mark) etc.).

Injections may comprise additional compound other than inert diluents: e.g. preserving agents, wetting agents, emulsifying agents, dispersing agents, stabilizing agent (such as human serum albumin, lactose, etc.), and assisting agents such as assisting agents for dissolving (arginine, asparaginic acid, etc.).

### Best Mode carrying out the Invention

The invention is illustrated by the following examples, but not limit the invention. In addition, unless described particularly in the following examples, the experiment was carried out by the method described in "Molecular Cloning" (Sambrook, J., Fritsh, E. F. and Maniatis, T., published on 1989 from Cold Spring Harbor Laboratory Press) or "Current Protocol in Molecular Biology" (F. M. Ausubel et al, published from John Wiley & Sons, Inc.). The procedure of kit etc. was in accordance with the method described in the attachment.

### Example

### [Preparation of cell]

First, undifferentiated mouse ES (Embryonic Stem) cell line D3 cells (See T. C. Doetschman, et. al., J. Embryol. Exp. Morphol., 87, 27, 1985) were maintained in the medium containing DMEM, 15% FCS, LIF on embryonic fibroblast cells treated with mitomycin C. ES cells (1 x 10⁵/well) treated with tripsin to become single cells were plated on the confluent mouse storomal cell line OP9 cells derived from op/op mouse neonatal calvaria (See H. Kodama, et. al., Exp. Hematol, 22, 979-984, 1994), and cultured in the medium containing a-MEM, 20% FCS at 37°C, under an atmosphere of 5% CO₂ for differentiation and induction into hematopoietic cells (See T. Nakano, et. al., Science, 265, 1098-1101, 1994). A half medium was exchanged at day 2 and all cells were recovered at day 5.

### [Preparation of poly (A) ⁺RNA]

Total RNA was extracted by using TRIzol Reagent (Trade mark, marketed by GIBCOBRL Co.). Poly (A) ⁺RNA was purified by Oligotex-dT30 〈Super〉 (Trade mark, marketed by Takara Shuzo).

### [Preparation of cDNA library of mammalian cell for signal sequence trap (SST)]

Using mRNA obtained above as a template, a single strand cDNAs were synthesized by reverse-transcriptase Super Script (Trade mark, marketed by GIBCOBRL Co.) with a random 9mer containing SalI site:
5'-GAGACGGTAATACGATCGACAGTAGGTCGACNNNNNNNNN-3' (SEQ ID NO. 16)
and double strand cDNAs were synthesized according to the method of Gublerr & Hoffman et al. Then, EcoRI adaptor:
5'-CCGCGAATTCTGACTAACTGATT-3' (SEQ ID NO. 17)
and
3'-CAGGCGCTTAAGACTGATTGACTAA-5' (SEQ ID NO. 18)
were ligated into the both ends of double strand cDNA by using DNA ligation kit Ver. 2 (Trade name, marketed by Takara Shuzo). 400∼600 bp cDNA fragments were separated by agarose-gel electrophoresis. Such obtained cDNA fragments were amplified by PCR (95°C for 30 seconds, 55°C for 1 minute, 72°C for 1 minute, 25 cycles) using a forward primer:
5'-CCGCGAATTCTGACTAACTGATT-3' (SEQ ID NO. 19)
and a reverse primer:
5'-GACGGTAATACGATCGACAGTAGG-3' (SEQ ID NO. 20)
After digestion with EcoRI and SalI, 400∼600bp cDNA fragments were separated by agarose-gel electrophoresis, and were ligated to EcoRl/SalI site of the pUC-SRa-Tac (described in the specification of International Patent publication No. 96/01843) and were transformed into E. coli DH5a by pUC-SRa-Tac to obtain a mammalian SST cDNA library.

### [Screening by SST]

Screening by SST was carried out according to the method described in Japanese Patent Application Kokai Hei 6-315380. Total 4,500 colonies of the cDNA library were divided into 125 pools (about 36 colonies/pool). The plasmids of each pool were prepared and were transfected into Cos 7 cell by DEAE-dextran method. After 48 hours, cells were separated from dish. Tac proteins on the cell surface were microscopically detected by immunostaining with fluoresene · isothiocyanate labeled anti-human Tac antibody (FITC-conjugated Monoclonal Mouse Anti Human Interleukin-2 Receptor Clone ACT-1, marketed by DAKO) to select the positive pools. In addition, colonies of the positive pools were divided into again. The same procedure was repeated until a single clone was obtained.

### [Determination of the nucleotide sequence of SST positive clone]

The nucleotide sequences of cDNA inserts in the positive clones were determined according to dye terminator cycle sequencing reaction using DNA Sequencing kit (Dye Terminator Cycle Sequencing Ready Reaction) (Trade name, marketed by Applied Biosystems Inc.) and analysis using automatic cDNA Sequencer-373 (Applied Biosystems Inc.). (Every nucleotide sequence was determined by this method hereinafter). The homology search for the obtained nucleotide sequences in data base revealed that 7 clones of cDNAs encoded known proteins containing signal sequence (secretary proteins such as Apolipoprotein E, Osteopontin etc. and membrane proteins such as PTPase-kappa etc.) and 17 clones of cDNAs were unknown.

### [Cloning full length cDNA and determination of nucleotide sequence]

Full length cDNA of a clone containing insert length of 444bp named as OHP106 out of the new 17 clones was isolated with 3'RACE (Rapid Amplification of cDNA End) in accordance with the method of 3'RACE System (Trade name, marketed by GIBCOBRL Co.). A single strand cDNA was synthesized by using the said total mRNAs as a template with 3'RACE adapter primer:
5'-GGCCACGCGTCGACTAC (T) 17-3' (SEQ ID NO. 21).
Using single strand cDNA as a template, PCR was carried out with OHP106 F2 primer:
5'-CTCCCATATTGACTGAATCTGAGAAGC-3' (SEQ ID NO. 22)
and Universal Amplification primer:
5'-GGCCACGCGTCGACTAC-3' (SEQ ID NO. 23)
at 95°C for 30 seconds, at 60°C for 30 seconds, at 72°C for 1 minute per cycle, total 30 cycles. In addition, using this PCR solution as a template, PCR was carried out again with OHP106F1 primer:
5'-AGTGGTTCTGCAACTCCTCC-3' (SEQ ID NO. 24)
and Universal Amplification primer:
5'-GGCCACGCGTCGACTAC-3' (SEQ ID NO. 25)
under the same condition to confirm that about 530bp cDNA was amplified specifically. These cDNA fragments were ligated to the pGEM-T (Trade name, marketed by Promega), and transfected into E. Coli DH5a to prepare plasmids. The determination of nucleotide sequences confirmed that the sequence of F1 primer and that of poly (A) ⁺ were contained at 5'-end and at 3'-end respectively. And then, full length cDNA sequence shown in SEQ ID NO. 3 was determined. In addition, open-reading frame was determined to obtain the nuclotide sequence shown in SEQ ID NO. 2 and deduced amino acid sequence shown in SEQ ID NO. 1.

It was indicated from the results of homology search for the public database of the nucleic acid sequences by using BLASTN and FASTA, and for the public database of the amino acid sequences by using BLASTX, BLASTP and FASTA, that there was no sequence identical to the polypeptide sequence and the nucleotide sequence of mouse OHP106 of the present invention. It was also indicated from the hydrophobisity analysis that the polypeptides of the present invention had no transmembrane region. Taken altogether, it was proved that polypeptides of the present invention were new secretary proteins. However, the search using BLASTX, BLASTP and FASTA revealed a significant homology between mouse OHP106 (region of 25th∼ 153th amino acid in SEQ ID NO. 1) and Chicken MD-1 (region of 28th∼154th amino acid in Pir Accession S42854) (See O. Burk et. al., EMBO J, 10, 3713-3719) of which the expression was induced by oncogene myb derived from Chicken leukemia virus. Based on these homologies, mouse OHP106 and chicken MD-1 were expected to share at least some activity.

### [Northern analysis]

Poly (A) ⁺RNA prepared from the undifferentiated ES cells and the differentiation and inducing ES cells were subjected to 1% agarose-gel electrophoresis and transferred onto a nylon membrane. Then, mouse OHP106 cDNA was labeled with ³²P-dCTP by using Random Primer DNA Labeling kit (Trade name, marketed by Takara Shuzo), and was hybridized with the membrane filter in 50% formamide, 5 x SSPE 0.1 % SDS, 2 x Denhaldts, 0.1mg/ml salmon sperm DNA at 42°C for 15 hours. After removing free ³²P-dCTP by 0.2 x SSC, 0.1% SDS at 65°C the membrane filter was autoradiographed for 48 hours and the analysis was carried out using BAS2000 (Fuji film). Northern analysis revealed that a band at near 600bp was found in the mRNA derived from the differentiation and inducing ES cells. From this result, it proved that the cDNA shown in SEQ ID NO. 3 was almost full length and that mRNA of mouse OHP106 was expressed on differentiation and inducing.

### [Determination of nucleotide sequence of gene related to mouse OHP106]

The clone (clone ID 520548) isolated from a mouse kidney cDNA library was purchased from IMAGE Consortium and named mouse OHP106K. This clone contained a 22bp insertion in the open reading frame of mouse OHP106. The nucleotide sequence of the full length clone was determined by the same method as mouse OHP106 to obtain the sequence shown in SEQ ID NO. 6. In addition, an open reading frame was determined to obtain the nucleotide sequence and deduced amino acid sequence shown in SEQ ID NOS. 5 and 4, respectively.

The clone (Clone ID 489463) isolated from a human pregnant uterus cDNA library was purchased from IMAGE Consortium Co. and named human OHP106. This clone had a homology to a part of mouse OHP106. The nucleotide sequence of the full length clone was determined by the same method as mouse OHP106 to obtain the sequence shown in SEQ ID NO. 9. In addition, an open reading frame was determined to obtain the nucleotide sequence and deduced amino acid sequence shown in SEQ ID NOS. 8 and 7, respectively.

Further, the clone (Clone ID 402964) isolated from a mouse aged 13.5-14.5 days embryo cDNA library and the clone (Clone ID 111816) isolated from a human fetus liver and kidney cDNA library were purchased from IMAGE Consortium Co. and named mouse OHP106H and human OHP106H, respectively. These clones encoded the polypeptides having a significant homology with a part of amino acid sequence of mouse OHP106. The nucleotide sequences of full-length clones were determined by the same method as mouse OHP106 to obtain the sequences shown in SEQ ID NOS. 12 and 15. In addition, an open reading frame was determined to obtain the nucleotide sequences shown in SEQ ID NOS. 11 and 14 and deduced amino acid sequences shown in SEQ ID NOS. 10 and 13. The homology search showed that the nucteotide sequences and putative amino acid sequences in coding regions of human and mouse OHP106 were 76% and 64% identical respectively. And the nucleotide sequences and putative amino acid sequences in coding regions of human and mouse OHP106H were 77% and 67% identical respectively. In addition, the nucleotide sequences and putative amino acid sequences of human OHP106 and human OHP106H were 49% and 23% identical respectively.

### [Expression of mouse OHP106 fusion protein using mammalian cells]

Using Xbal-mouse OHP106F primer including a initiation ATG codon of OHP106:
5'-CGTCTAGACGGAGATATTAAATCATGTTGC-3' (SEQ ID NO. 26)
and Xbal- FLAG-mouse OHP106H primer including a termination TGA codon: 
5'-CGTCTAGATCACTTGTCATCGTCGTCCTTGTAGTCATTGACATCA
CGGCGGTGAATGAT-3'    (SEQ ID NO. 27)
 or the mentioned Xbal-mouse OHP106F primer and Xbal-6xHis-mouse QHP106H primer including a termination TGA codon: 
5'-CGTCTAGATCAGTGATGGTGATGGTGATGATTGACATCACGGCG
GTGAATGAT-3'   (SEQ ID NO. 28),
 PCR was performed with a single strand cDNA prepared at 3'RACE as a template. The amplified cDNA fragments were digested by Xbal and cloned in Xbal site of pEF-BOS expression vector of mammalian cells (S. Mizushima et. al. Nucleic Acid Res., 18, 5322) to express the FLAG-OHP106 or 6-His-OHP106 fusion proteins. FLAG (polypeptide consisting of AspTyrLys AspAspAspAspLys) or six His residues were incorporated at the C-terminal of the OHP106 protein as tags. Thus, the plasmids were prepared, and their nucleotide sequences were confirmed.

Thus obtained plasmids were transfected into Cos 7 cell using Lypofectine (Trade name, marked from GIBCOBRL). After 24 hours, the transfection mixture was removed. The cells were cultured in the medium without serum for 72 hours. The supernatants were harvested and 10-fold concentrated with Centricon-10 (Trade name, marketed by Amicon). The samples were separated on SDS-PAGE gels, and transferred to Imobiron-P (PVDF membrane, Trade name, marketed by Milypore Co.). The expression of the mouse OHP106-FLAG fusion protein and the mouse OHP106-6xHis fusion protein were detected by the immunobloting using anti-FLAG M2 monoclonal antibody (marketed by Eastman Kodak Co.) and horseradish peroxidase conjugated protein A and using horseradish peroxidase conjugated Ni-NTA (Trade name, marketed by QIAGEN) respectively.

### [Expression of the mouse OHP106, protein and their related proteins (abbreviated as mouse OHP106 etc. hereinafter) using mammalian cell]

Thus obtained full length cDNAs encoded mouse OHP106 etc. were cloned in XhoI (or EcoRI)-NotI site of the pED6 expression vector of mammalian cells (See Kaufman et al., Nucleic Acids Res. 19, 4485-4490 (1991)) to express the mouse OHP106 etc. The obtained plasmids were transfected into Cos 7 cells using Lypofectine (Trade name, marketed by GIBCOBRL). After 24 hours, the transfection mixture was removed. The cells were cultured in the Met and Cys-free medium with ³⁵S-labeled Met and ³⁵S-labeled Cys for 5 hours. The supernatants were harvested and 10-fold concentrated with Centricon-10 (Trade name, marketed by Amicon). The samples were separated on SDS-PAGE gels. After drying the gel, the expression of the mouse OHP106 etc. were detected using BAS2000. That is to say, the dried gels were exposed with the Imaging · Plates (Fuji Imaging · Plate TYPE-III), analyzed by using Imaging · Analyzer (Fuji Imaging · Analyzer BAS2000 System) and printed out to printer (Fuji Pictrography 3000). For example, the expression of mouse OHP106H protein was shown in Figure 1. A single band of approximately 20kDa was detected in the supernatant from the cells transfected with mouse OHP106H cDNA, but not in supernatant from the cells transfected with pED6 vector alone. This result confirmed the expression of the mouse OHP106H protein.

## Claims

1. Substantially purified form of the polypeptide that comprising the amino-acid sequence shown in SEQ ID NOS. 1, 4, 7, 10 or 13, homologue thereof, fragment thereof or homologue of the fragment.

2. A polypeptide according to claim 1 that comprising the amino-acid sequence shown in SEQ ID NOS. 1, 4, 7, 10 or 13.

3. A cDNA encoding the polypeptide according to claim 1.

4. A cDNA according to claim 3 that comprising the nucleotide sequence shown in SEQ ID NOS. 2, 5, 8, 11 or 14 a fragment cDNA selectively hybridized to the cDNA.

5. A cDNA according to claim 3 that comprising the nucleotide sequence shown in SEQ ID NOS. 3, 6, 9, 12 or 15, or a fragment cDNA selectively hybridized to the cDNA.

6. A replication or expression vector carrying the cDNA according to claims 3 to 5.

7. A host cell transformed with the replication or expression vector according to claim 6.

8. A method for producing the polypeptide according to claim 1 or 2 which comprises culturing a host cell according to claim 7 under a condition effective to express the polypeptide according to claim 1 or 2.

9. A monoclonal or polyclonal antibody against the polypeptide according to claim 1 or 2.

10. A pharmaceutical composition containing the polypeptide according to claim 1 or 2 or the antibody according to claim 9, in association with pharmaceutically acceptable diluent and/or carrier.
